# EUROPÄISCHE PATENTSCHRIFT

(11) **EP 1 015 873 B1**
(45) Veröffentlichungstag und Bekanntmachung des Hinweises auf die Patenterteilung: **20.10.2004**
(21) Anmeldenummer: 97933612.0
(22) Anmeldetag: 14.08.1997
(51) Int. Cl.: G01N 21/89, G01N 33/36, G01B 11/10, G01B 11/30

(54) **VORRICHTUNG ZUR OPTISCHEN ERFASSUNG VON EINEM PARAMETER AN EINEM LÄNGSBEWEGTEN FADENFÖRMIGEN KÖRPER**
DEVICE FOR OPTICAL CAPTURE OF A PARAMETER OF A LONGITUDINALLY MOVING THREAD-SHAPED BODY
DISPOSITIF DE DETECTION OPTIQUE D'UN PARAMETRE SUR UN CORPS FILIFORME SE DEPLA ANT DANS LE SENS LONGITUDINAL

(30) Priorität: 20.08.1996 CH 203096
(43) Veröffentlichungstag der Anmeldung: 05.07.2000
(73) Patentinhaber: Uster Technologies AG, 8610 Uster (CH)
(72) Erfinder: HENSEL, Rolf, CH-8008 Zürich (CH); WAMPFLER, Hans, CH-8049 Zürich (CH); RAYNOR, Jeffrey, Mitchell, Edimburgh, EH12 5BD (GB); SEITZ, Peter, Markus, CH-8902 Urdorf (CH)
(74) Vertreter: Ellenberger, Maurice
(86) Internationale Anmeldenummer: PCT/CH1997/000300
(87) Internationale Veröffentlichungsnummer: WO 1998/008079

(56) Entgegenhaltungen:
- EP-A- 0 553 446
- CH-A- 643 060
- DE-A- 4 131 664

## Beschreibung

Die Erfindung betrifft eine Vorrichtung zur optischen Erfassung von mindestens einem Parameter an einem längsbewegten fadenförmigen Körper.

Aus der CH 643 060 ist ein Verfahren und eine Vorrichtung zur Bestimmung des Durchmessers oder des Querschnitts eines faden- oder drahtförmigen Körpers bekannt. Dabei wird der Schattenwurf des von einer Lichtquelle angestrahlten Körpers auf einem Bildaufnehmer ausgemessen, der aus einer Reihe nebeneinander angeordneter Fotoelementen besteht. Die Fotoelemente geben impulsförmige Signale ab, die zusammen in einem Auswertegerät ausgewertet und in konkrete Durchmesser- oder Querschnittswerte umgeformt werden.

Ein Nachteil dieses bekannten Verfahrens ist darin zu sehen, dass für gewisse Parameter genaue Messresultate mit entsprechend hohem Schaltungsaufwand erreicht werden müssen, denn, beispielsweise brauchbare Haarigkeitswerte für ein Garn lassen sich damit beispielsweise nur erreichen, wenn die einzelnen Fotoelemente kleine Dimensionen aufweisen und in entsprechend grosser Zahl vorgesehen sind.

Die Erfindung, wie sie in den Patentansprüchen gekennzeichnet ist, löst nun die Aufgabe, eine Vorrichtung zu schaffen, mit der Parameter wie der Durchmesser eines fadenförmigen Körpers, der Durchmesser eines Garnkörpers, die Haarigkeit eines Garns usw. einfacher und genauer ermittelt werden können.

Die Aufgabe wird durch eine Vorrichtung gemäß Anspruch 1 gelöst. Somit weist der Sensor einerseits Einzelsensoren auf, die beispielsweise einen Parameter wie den Durchmesser des Körpers direkt digital erfassen und er weist andererseits einen Einzelsensor auf, der denselben oder einen anderen Parameter analog erfasst. Der Sensor weist demnach Einzelsensoren auf, die nach unterschiedlichen Prinzipien arbeiten oder deren Signale nach unterschiedlichen Prinzipien ausgewertet werden. Dieser optische Sensor hat vorzugsweise eine Ausdehnung, die diejenige des Körpers quer zu seiner Längsrichtung übersteigt und ist vorzugsweise so ausgebildet, dass die Erfassung eines Parameters mindestens teilweise an demselben Bereich des Körpers erfolgt. Die Sensoren sollen durch gerichtetes Licht beleuchtet werden, so dass das Garn zwischen der Lichtquelle und dem Sensor das Licht abschattet. Der Sensor ist mit einer Auswerteschaltung verbunden, mit der auch die Signale aus mehreren Einzelsensoren gemeinsam ausgewertet werden können.

Die durch die Erfindung erreichten Vorteile sind insbesondere darin zu sehen. dass neben dem Durchmesser eines vergleichsweise glatten Körpers auch der Durchmesser eines Körpers mit aufgelöster Oberflächenstruktur in differenzierter Weise gemessen werden kann, ohne eine Vorrichtung zu benötigen, die sehr aufwendig gestaltet ist. Beispielsweise können an einem Garn der Garnkörper (ohne abstehende Fasern) und die Haarigkeit (Anteil abstehender Fasern) des Garns getrennt gemessen werden. Ein solcher Sensor kann durch eine Auswerteelektronik auch an veränderte Messbedingungen angepasst werden und beispielsweise den Einfluss von Schmutz und Ablagerungen auf den Sensoren ausgleichen oder berücksichtigen.

Im folgenden wird die Erfindung anhand eines Beispiels und mit Bezug auf die beiliegenden Zeichnungen näher erläutert. Es zeigen:
Figuren 1 bis 3 je einen Teil der erfindungsgemässen Vorrichtung in vereinfachter Darstellung,
Figuren 4 bis 7 je einen weiteren Teil der Vorrichtung,
Figur 8 eine schematische Darstellung einer Funktion eines Teils der Vorrichtung
Figur 9 ein analoges und ein digitales Signal,
Figur 10 einen Querschnitt durch einen fadenförmigen Körper mit möglichen Abmessungen und
Figuren 11 und 12 je ein Signal aus der Vorrichtung.

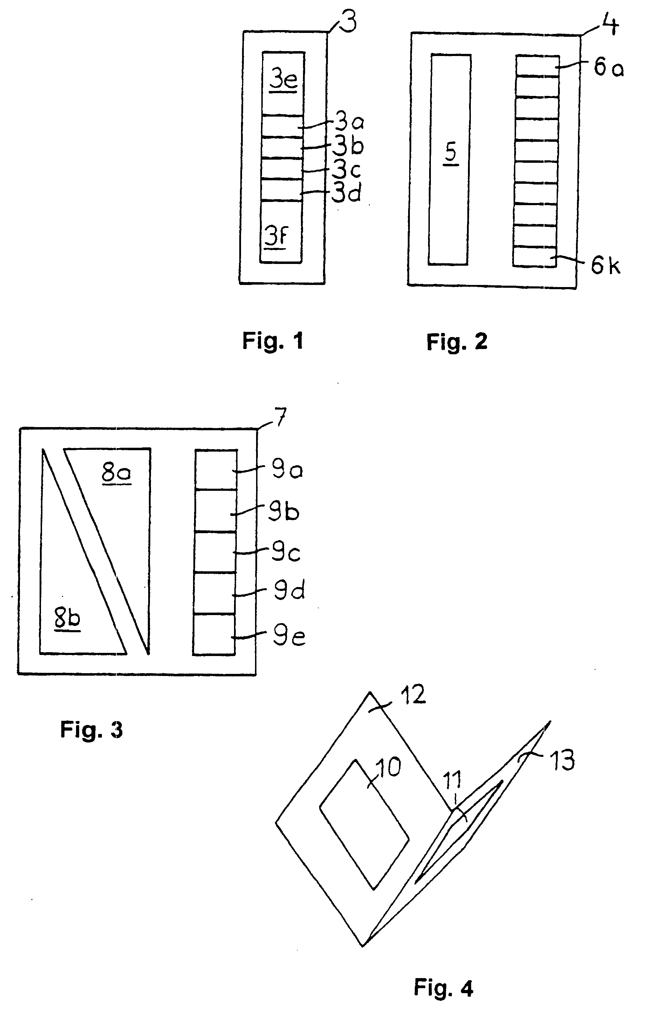

Fig. 1 zeigt einen Sensor 3 mit Einzelsensoren 3a, 3b, 3c, 3d sowie 3e und 3f, die in einer Reihe angeordnet sind. Hier können beispielsweise die Einzelsensoren 3a - 3d digital, die Einzelsensoren 3e und 3f dagegen analog arbeiten. So ergibt sich ein Sensor 3 mit Einzelsensoren 3a, 3e die nach verschiedenen Prinzipien arbeiten, mindestens was die Verarbeitung der Signale betrifft, die sie abgeben. Die Einzelsensoren sind in Richtung des zu messenden Parameters angeordnet, also in Richtung des Durchmessers oder Querschnittes des Körpers K.

Fig. 2 zeigt einen Sensor 4 mit Einzelsensoren 5 und 6a - 6k. Hier kann beispielsweise der Einzelsensor 5 analog arbeiten und die Einzelsensoren 6a-k arbeiten zusammen digital, indem die Einzelsignale zu einem digitalen Signal zusammengesetzt werden.

Fig. 3 zeigt einen weiteren Sensor 7 mit Einzelsensoren 8a, 8b und 9a - 9e. Wie auch beim Sensor 4, (Fig. 2) decken die Einzelsensoren 8 und 9 vorzugsweise dieselbe Messfeldhöhe ab, oder sind mindestens teilweise demselben Bereich des Körpers (hier in y-Richtung) zugeordnet.

Fig. 4 zeigt Sensoren 10 und 11, die in zwei zueinander geneigten Ebenen 12 und 13 angeordnet sind.

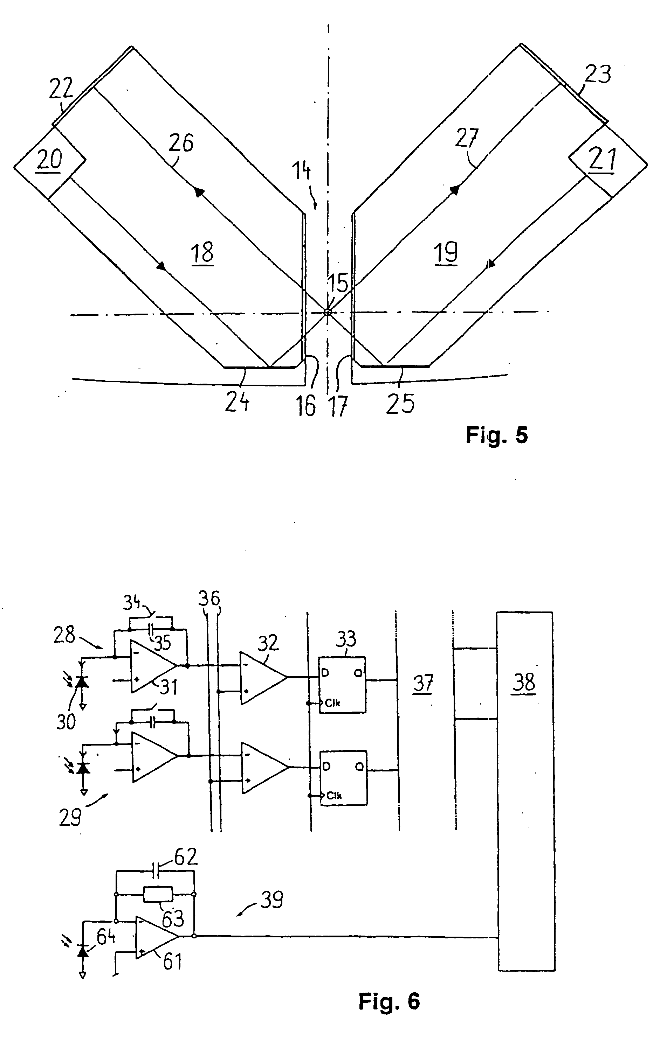

Fig. 5 zeigt einen Messspalt 14 wie er üblicherweise zum Messen oder Überwachen von Garn 15 verwendet wird. Der Messspalt 14 wird durch je ein Deckglas 16, 17 seitlich begrenzt. Jenseits der Deckgläser 16, 17 erstrecken sich Lichtschächte oder Lichtleiter 18, 19, die je zu einem Sender 20, 21 und einem Empfänger 22, 23 für Lichtsignale führen. Die Lichtleiter 18, 19 weisen je einen Spiegel 24, 25 auf, so dass sich zwei Strahlengänge 26, 27 ergeben, die jeweils vom Sender 20, 21 über die Spiegel 24, 25 und das Garn 15 zum Empfänger 23, 22 führen. Die Sender 20, 21 sind vorzugsweise so ausgebildet, dass sie im wesentlichen gerichtetes Licht aussenden. Die Empfänger 22, 23 weisen vorzugsweise eine telezentrische Empfangsoptik auf. Damit treten im Bereiche der Deckgläser 16, 17 und des Messspaltes 14 im wesentlichen parallele Lichtstrahlen auf. So kann sich die Position des Garnes 15 im Messspalt 14 ändern, ohne dass dies die Grösse der Abbildung des Garnes 15 auf die Empfänger 22, 23 ändert. Der Massstab bleibt somit gleich. Durch diese Anordnung ist auch gewährleistet, dass sich die orthogonalen Lichtstrahlen im Messspalt 14 kaum beeinflussen und damit simultan für die Gewinnung von Messwerten eingesetzt werden können.

Fig. 6 zeigt zwei je gleich aufgebaute Schaltungen 28 und 29 wie sie für jeden Einzelsensor vorgesehen sein können. Eine solche besteht aus einem Element 30 zur Umwandlung von Licht in einen elektrischen Strom, beispielsweise einer Photodiode. Dieses Element 30 gilt vorzugsweise als eigentlicher Einzelsensor. Dieser ist mit weiteren Elementen zur Umwandlung seines Ausgangssignales, wie einem Ladungsverstärker 31, 35, einem Komparator 32 und einem Speicher oder Latch 33 in Serie geschaltet. Der Ladungsverstärker 31, welcher aus einem Operationsverstärker 31 und einem Kondensator 34 (im Feedbackpfad) besteht, ist weiter mit einem Schalter 34 parallel geschaltet. Der Komparator 32 ist mit einem Eingang an eine Referenzleitung 36 angeschlossen. Der Speicher 33 ist mit seinem Ausgang an einen Multiplexer 37 angeschlossen. Dem Multiplexer 37 nachgeschaltet ist wiederum eine Auswerteschaltung 38, welche vorzugsweise als Rechner ausgebildet sein kann. Ebenfalls an die Auswerteschaltung 38 angeschlossen ist wahlweise eine Schaltung 39 zur Erzeugung eines analogen Einzelsignales. Diese weist neben einem Einzelsensor 64 insbesondere einen Operationsverstärker 61 mit einem parallel geschalteten Kondensator 62 und einem Widerstand 63 auf. Vorzugsweise ist mindestens ein Teil der vorgenannten Elemente mit dem Einzelsensor 30, 64 zu einer integrierten Schaltung integriert und bilden damit einen sogenannten "smart sensor".

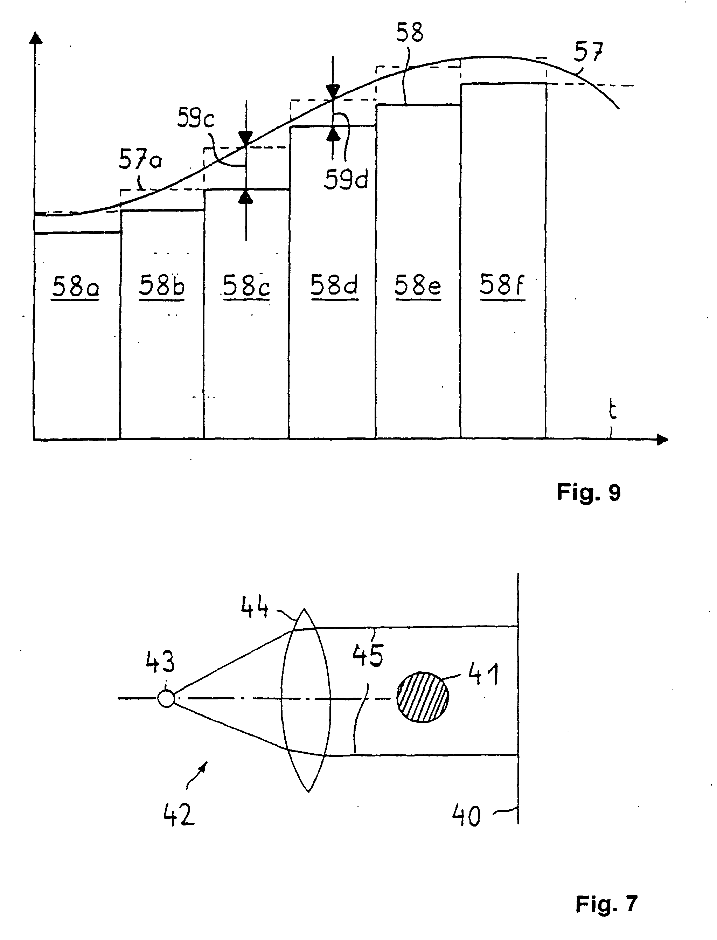

Fig. 7 zeigt schematisch eine Oberfläche 40 eines der Sensoren, einen fadenförmigen Körper 41 im Schnitt und eine Lichtquelle 42 für gerichtetes Licht. Diese kann beispielsweise aus einer punkt- oder zeilenförmigen Lichtquelle 43 und einer telezentrischen Optik 44 bestehen. Damit können gerichtete und vorzugsweise parallele Strahlen 45 erzeugt werden.

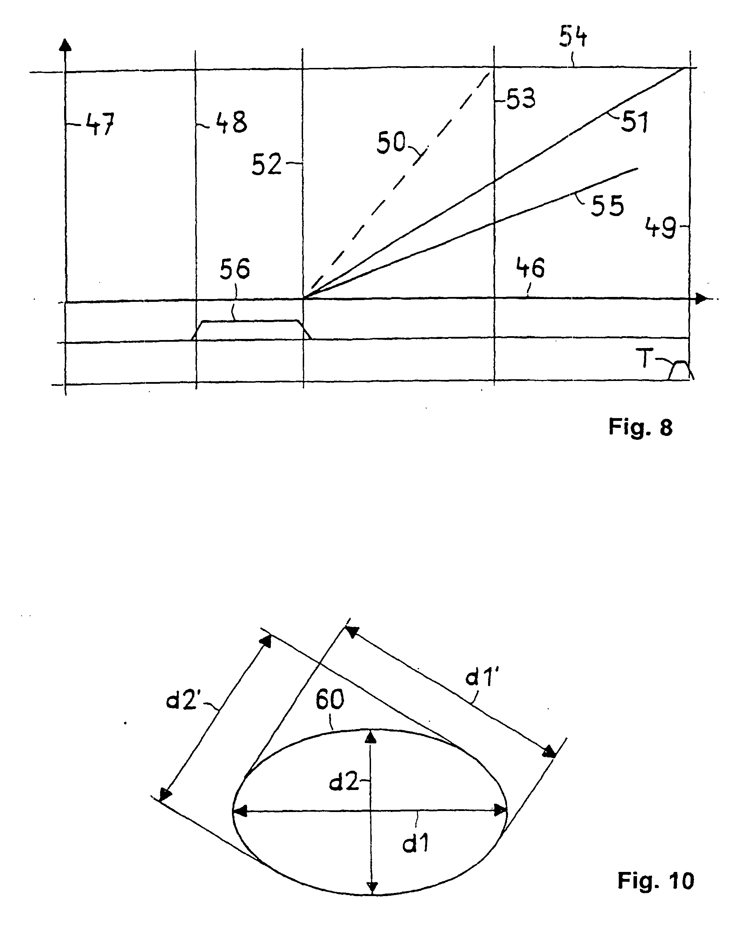

Fig. 8 zeigt verschiedene Linien, die Vorgänge in der Vorrichtung betreffen und die über einer Zeitachse 46 und neben einer Achse 47, längs der Werte für elektrische Spanungen oder Prozentzahlen möglicher Werte davon aufgetragen sind, dargestellt sind. Linie 48 markiert beispielsweise den Anfang und Linie 49 das Ende eines periodischen Zyklus. Linien 50, 51 und 55 zeigen die Aufladung des Kondensators 35 mit der Zeit in verschiedenen Situationen an, die mit einer Zeit entsprechend einer Linie 52 beginnen.

Fig. 9 zeigt vereinfacht über einer Zeitachse t aufgetragen ein analoges Einzelsignal 57 und ein digitalisiertes und getaktetes Einzelsignal 58, das aus einzelnen Werten 58a bis 58f besteht. Durch die unterschiedlichen Prinzipien, die bei der Messung oder Auswertung verwendet werden, ergeben sich beispielsweise Differenzwerte 59c, 59d usw. zwischen dem analogen und dem digitalen Signal.

Fig. 10 zeigt eine Kontur 70 eines Querschnittes eines fadenförmigen Körpers, die hier unrund und insbesondere ellipsenförmig angenommen ist. d1 und d2 sind Hauptabmessungen, wie sie längs der Hauptachsen der Kontur 60 ermittelt werden, d1' und d2' sind Hauptabmessungen, wie sie in zwei anderen, orthogonalen Richtungen ermittelt werden.

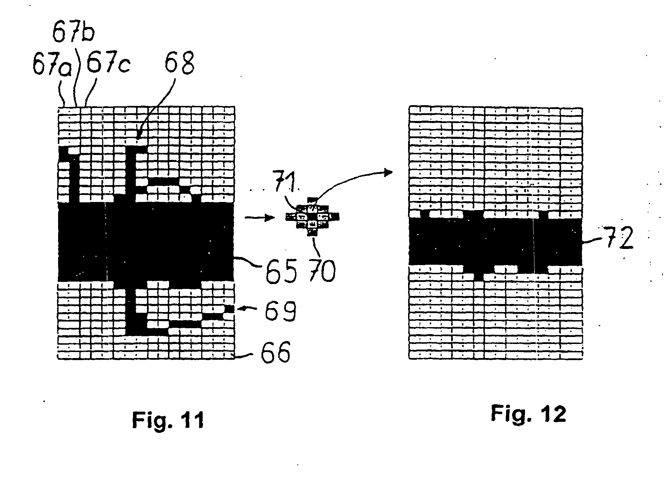

Fig. 11 zeigt eine Darstellung eines fadenförmigen Körpers 65, wie sie durch Einzelsensoren 6 (Fig. 3) mit entsprechender Auflösung beispielsweise in der Auswerteschaltung 38 entsteht, in der mehrere aufeinaderfolgende Messzyklen gespeichert sind. Hier haben die Einzelsensoren, wie z.B. der Einzelsensor 66 kleinere Abmessungen als die Einzelsensoren 6 und 9 und jedem Einzelsensor oder Pixel entspricht in einem Speicher der Auswerteschaltung 38 ein Speicherplatz, der mit einem binären Signal belegt ist. Um eine solche Darstellung zu erhalten, sind mehrere Kolonnen 67a, 67b, 67c usw. gespeichert wobei jede Kolonne 67 zu einem bestimmten Messzyklus gehört. Neben dem eigentlichen Körper 65 und davon abstehend, sind einzelne Fibrillen oder Fasern zu erkennen, die mit 68 und 69 bezeichnet sind. Mit 70 ist eine sogenannte Erosionsmatrix bezeichnet, die für die Durchführung an sich bekannter und sogenannter Nachbarschaftsoperationen verwendet wird. Diese besteht hier aus dreizehn Pixeln oder Speicherplätzen, die um ein zentrales Pixel 71 angeordnet sind, auf welches die Nachbarschaftsoperation ausgeübt wind.

Fig. 12 zeigt eine Darstellung entsprechend Fig. 11, bei der die abstehenden Fibrillen oder Fasern durch die Nachbarschaftsoperation weg-erodiert sind. So erkennt man nur noch ein grossflächiges Gebilde wie den eigentlichen Körper 72, dessen Durchmesser durch die Erosion um etwa zwei Pixel auf jeder Seite künstlich verkleinert ist.

Die Wirkungsweise der Vorrichtung ist wie folgt:

Wie in Fig. 7 dargestellt, wird ein fadenförmiger Körper 41 wie z.B. ein Garn, eine Faser, ein Draht usw., wie dies beispielsweise bei bekannten Garnprüfgeräten und Garnreinigem der Fall ist, in einem Messspalt in seiner Längsrichtung an einem Sensor vorbeibewegt, dessen Oberfläche 40 hier dargestellt ist. Die Oberfläche 40 wird durch den Körper 41 gegenüber der Lichtquelle 42 abgedeckt oder abgeschattet. Hinter der Oberfläche 40 ist ein Sensor 1, 3, 4 oder 7 vorgesehen, wie er aus einer der Figuren 1 bis 3 bekannt ist.

Mit dem Sensor 3 (Fig. 1) kann als Parameter der Durchmesser eines Körpers erfasst werden . Geht man davon aus, dass die Einzelsensoren 3a - 3d Einzelsignale abgeben, die binär erfasst werden und dass die Einzelsensoren 3e und 3f Einzelsignale abgeben, die analog erfasst und weiterverarbeitet werden, so kann damit eine differenzierte Erfassung von Randbereichen des Körpers ermöglicht werden. Oder es kann mit den Einzelsensoren 3a - 3d der Durchmesser und mit den Einzelsensoren 3e und 3f das Vorhandensein abstehender Teile und deren ungefähre Ausmasse erfasst werden.

Mit dem Sensor 4 (Fig. 2) kann der Durchmesser des Körpers einerseits digital durch die Einzelsensoren 6a - 6k und andererseits analog durch den Einzelsensor 5 erfasst werden. Der Einzelsensor 5 liefert ein Einzelsignal, das proportional zur Abschattung durch den Körper ist. Die Einzelsensoren 6 liefern je ein Einzelsignal, das zwar ebenfalls proportional zur Abschattung ist, das aber binarisiert ist, so dass aus den Einzelsignalen der Einzelsensoren 6 ein digitales Signal erzeugt wird. Durch Vergleich des Einzelsignals aus dem Einzelsensor 5 und des Signals aus den Einzelsensoren 6 können weitere Parameter wie z.B. die Haarigkeit, Struktur usw. des Körpers bestimmt werden, insbesondere dann, wenn der Körper ein Garn ist.

Mit dem Sensor 7 (Fig. 3) kann im Prinzip gleich gemessen werden wie mit dem Sensor 4, mit dem Unterschied, dass die Einzelsensoren 8 je ein Einzelsignal abgeben, das von der Lage des Körpers vor den Einzelsensoren 8 in y-Richtung abhängig ist. Ist namlich der Körper am unteren Rand des Sensors 7, so schattet er vorwiegend den Einzelsensor 8b ab, so dass das Einzelsignal des Einzelsensors 8b viel stärker beeinflusst ist als das Einzelsignal des Einzelsensors 8a. Ist der Körper am oberen Rand des Sensors 7, so ist der Einzelsensor 8a stärker beeinflusst.

Mit einer Vorrichtung gemäss Fig. 4, bei der Sensoren 10 und 11 in zwei Ebenen 12, 13 angeordnet sind, lässt sich der Körper aus zwei Richtungen betrachten, was genauere Rückschlüsse auf den wahren Querschnitt des Körpers zulässt. Als Sensoren 10, 11 sind Sensoren 1, 3, 4, 7 oder andere vorgesehen.

Mit der Vorrichtung gemäss Fig. 5 lässt sich ein Körper, hier ein Garn 15 ebenfalls aus zwei Richtungen, entsprechend den Strahlengängen 26, 27, betrachten. Der Sender 20 sendet einen Lichtstrahl auf den Spiegel 24, der von dort auf den Empfänger 23 geleitet wird. Dabei schattet das Garn 15 den Empfänger 23 ab, der aus einem der Sensoren 1, 3, 4, 7 besteht. Der Sender 21 sendet einen Lichtstrahl auf den Spiegel 25, der von dort auf den Empfänger 22 geleitet wird. Dabei schattet das Garn 15 den Empfänger 22 ab, der aus einem der Sensoren 1, 3, 4, 7 besteht. Als Lichtstrahl ist hier ein ganzes Bündel vorzugsweise gerichteter und paralleler Strahlen zu verstehen, so dass das Garn 15 auch dann erfasst wird, wenn es sich nicht genau an der gezeigten Stelle im Messspalt 14 befindet.

Wird nun ein Einzelsensor teilweise oder ganz von einem Körper gegenüber einer Lichtquelle abgedeckt, so läuft dabei ein Zyklus etwa folgendermassen ab. Zu einer Zeit 48 (Fig. 8) startet dieser Zyklus damit, dass ein Rücksetzsignal 56 ausgelöst wird, das den Schalter 34 (Fig. 6) schliesst, geschlossen hält und bis zu einer Zeit 52 dauert, mit der damit begonnen wird, den oder die Kondensatoren 35 durch Photoströme aus dem oder den Einzelsensoren aufzuladen, oder, anders ausgedrückt, das erfasste Signal zu integrieren.

Ist ein Einzelsensor vom Körper 41 gegenüber der Lichtquelle 42 nicht abgedeckt, so geht die Aufladung des Kondensators 35 schnell vor sich, wie dies die Linie 50 zeigt, und ist zur Zeit 53 abgeschlossen, wenn eine Schwelle 54 erreicht ist. Der Operationsverstärker 31 verstärkt dabei das Signal aus dem Kondensator 35 und gibt es an den Komparator 32 ab. Dieser vergleicht laufend das Signal gemäss Linie 50 mit einem Schwellwert, der durch eine Linie 54 dargestellt ist und über eine Leitung 36 anliegt. Ist der Schwellwert 54 erreicht, so gibt der Komparator 32 ein Signal an den Speicher 33 ab, das angibt, dass der einzelsensor nicht abgedeckt ist. Dieses Signal hat lediglich zwei mögliche Werte und ist ein binäres Signal.

Ist ein Einzelsensor durch den Körper abgeschattet, so erhält er kein direktes Licht, sondern bestenfalls Streulicht. Damit lädt sich der Kondensator 35 nur mehr langsam, beispielsweise entsprechend einer Linie 55 auf und erreicht den Schwellwert 54 bestenfalls nach einer sehr langen Zeit, die die Zykluszeit übersteigt. Das erfasste Signal wird damit während einer vorgegebenen Zeit integriert und dann zurückgesetzt. Der Speicher 33, der mit derselben Zykluszeit arbeitet und damit synchron zum Schalter 34 getaktet ist, erhält nun vom Komparator 32 ein Signal, das angibt, dass der Einzelsensor abgeschattet ist und dieses Signal kann zusammen mit den Signalen aus den Speichern der anderen Einzelsensoren ausgegeben werden. Aus allen Signalen stellt der Multiplexer 37, durch Aneinanderreihen der einzelnen binären Werte ein Signal her, das ein Bild über die Belichtung des ganzen Sensors ergibt. Daraus kann beispielsweise ein Wert für den Garnquerschnitt abgeleitet werden.

Die Zykluszeit wird durch die Linien 48 und 49 abgegrenzt. Je nach Qualität des Einzelsensors oder je nach Grad der Verschmutzung des Einzelsensors geht es mehr oder weniger lange bis die Aufladung des Kondensators 35 den Schwellwert 54 erreicht. Die Linien 49 bzw 51 geben an, wie lange die Aufladung des Kondensators 35 dauert. wenn nur 50% des möglichen Lichts den Einzelsensor erreicht. Es kann als Ansatz für die Bemessung der zulässigen Zeit für das Aufladen des Kondensators 35 davon ausgegangen werden, dass das Erreichen des Schwellwertes 54 mit halber Leistung noch innerhalb des Zyklus und damit der Zeit 49 möglich sein sollte. Durch Verschieben der Linen 52, 53 innerhalb der Zykluszeit kann dies durch Verlängern oder Verkürzen der Dauer des Rücksetzsignales 56 eingestellt werden, was auch bedeutet, dass das Rücksetzsignal 56 den übrigen Teil der Zeit im Zyklus beansprucht. Damit gelten Einzelsensoren, die sich in der Zeit zwischen den Linien 49 und 52 nicht genügend aufladen als vom Körper abgedeckt. Wenn die Verschmutzung unbedeutend ist und wenn es sich um einen besonders guten Einzelsensor handelt, können die Linien 52, 53 gegen die Linie 49 hin verschoben sein und die Linien 50, 51 steiler verlaufen. Diese Vorgänge können für jeden Einzelsensor nachgeführt werden wobei als Regelgrösse diejenige Zeit ermittelt wird, die benötigt wird, bis das Signal des ersten der beteiligten Einzelsensoren die Schwelle 54 erreicht hat. Diese Zeit gilt als Ist-Wert für die Regelung. Der verdoppelte Wert dieser Zeit ergibt die Belichtungs- oder Integrationszeit, die zwischen den Linien 49 und 52 liegt. Ist diese zu kurz, so erreicht der erste Einzelsensor die Schwelle 54 zu spät, d.h. erst nach mehr als der halben Zeit. Dann muss diese eben verlängert werden.

Geht man beispielsweise davon aus, dass mit dem Sensor 4 durch den Einzelsensor 5 und eine Schaltung 39 (Fig. 6) ein analoges Einzelsignal 57 (Fig. 9) erzeugt wird, das proportional zum Durchmesser des Körpers ist, und dass durch die Einzelsensoren 6a - 6k und Schaltungen 29, 30 usw. ein digitales Einzelsignal 58 erzeugt wird, das ebenfalls proportional zum Durchmesser des Körpers ist, so stellt man fest, dass beide Einzelsignale nicht genau übereinstimmen, auch wenn sie vom selben Körper stammen. Differenzwerte 59 ergeben sich beispielsweise daraus, dass die Einzelsensoren 5 und 6 Randbereiche des Körpers nicht gleich erfassen. Beispielsweise erfassen die Einzelsensoren 6 bei einem Garn eher den Garnkörper, wogegen der Einzelsensor 5 das Garn mit abstehenden Fasern erfasst. Die Differenzwerte 59 können beispielsweise bei einem Garn der Haarigkeit entsprechen und werden als solche im Rechner 38 aus den Einzelsignalen 57 und 58 durch Subtraktion ermittelt. Damit werden vom selben Körper zwei Signale parallel erfasst. Eines davon ist getaktet.

Mit der Vorrichtung gemäss Fig. 4 kann der Körper aus zwei Richtungen erfasst werden. Soll als Parameter der Querschnitt des Körpers ermittelt werden, so müssen dazu zwei verschiedene Durchmesser gemessen werden. Dazu gibt es verschiedene Möglichkeiten, wie dies die Fig. 10 zeigt. Es können als Durchmesser Hauptabmessungen d1 und d2 oder d1' und d2' ermittelt werden. Da mit der Vorrichtung gemäss Fig. 4 beispielsweise zwei Richtungen vorgegeben werden, die beispielsweise senkrecht zueinander stehen, bleibt die Ungewissheit darüber, welche Hauptabmessungen nun erfasst werden, denn dies hängt von der zufälligen Lage des Körpers ab. Um diesen Einfluss möglichst gering zu halten, sollen die beiden erfassten Hauptabmessungen zweimal verrechnet werden und zwar soll das Produkt d1*d2 oder d1'*d2' der Hauptabmessungen und die halbe Summe der Quadrate der Hauptabmessungen also 0.5(d1² + d2²) oder 0.5(d1'² + d2'²) gebildet werden. Dies kann in der Auswerteschaltung 38 durchgeführt werden, an die alle Einzeisensoren der Sensoren 10 und 11 angeschlossen sind. Aus den beiden Hauptabmessungen können auch Angaben über Parameter wie die Rundheit (Rundmass) des Körpers, bei Zwirnen über die Zwirndrehung, gewonnen werden, indem beispielsweise der Quotient aus dem kleinen Durchmesser und dem grossen Durchmesser, z.B. d2/d1 berechnet wird.

Vorzugsweise sind die integrierenden Einzelsensoren 5, 8, die ein analoges Einzelsignal abgeben, wie die Einzelsensoren 6, 9, die ein digitals Signal abgeben, mit dem selben Taktsignal getaktet. Dann ergibt dies in Fig. 9 ebenfalls eine Treppenkurve 57a, die das Einzelsignal 57 ersetzt. Trotzdem basiert aber die Treppenkurve 57a auf analog erfassten und verarbeiteten Signalen.

Die Einzelsignale aus den Einzelsensoren können wahlweise durch an sich bekannte Nachbarschaftsoperationen weiterbehandelt werden. Dabei werden zuerst die Resultate der digitalisierenden Einzelsensoren 6, 9 aus einigen aufeinanderfolgenden Zyklen gespeichert. Die Signale jedes Einzelsensors aus einem dieser Zyklen werden mit den benachbarten Signalen, d.h. den Signalen desselben Einzelsensors in benachbarten Zyklen und den Signalen benachbarter Einzelsensoren im selben und in benachbarten Zyklen erfasst und mit dem betreffenden Signal des Einzelsensors verglichen. Dadurch wird für jedes Einzelsignal auch die Umgebung gebildet und aus der Umgebung auffallende einzelne Signale eines Einzelsensors werden den umgebenden Signalen angepasst. Damit werden lockere Strukturen, die aus lose zusammenhängenden Pixeln bestehen weggelöscht und es bleiben nur grossflächige Gebilde, wie z. B. ein Garnkörper, übrig, wie dies die Fig. 12 zeigt.

## Patentansprüche

1. Vorrichtung zur optischen Erfassung von mindestens einem Parameter an einem längsbewegten fadenförmigen Körper (K) mit einem optischen Sensor (3, 4, 7), der aus mindestens zwei Einzelsensoren (3a, 3b, 6a, 6b, 9a, 9b) zusammengesetzt ist, wobei ein Messwert für den Parameter durch mindestens einen Einzelsensor digital erfassbar ist, **dadurch gekennzeichnet, dass** der Sensor mindestens einen weiteren Einzelsensor (3e, 3f, 5, 8a, 8b) zur analogen Erfassung eines Parameters aufweist.

2. Vorrichtung nach Anspruch 1, **dadurch gekennzeichnet, dass** mehrere Einzel sensoren (3a - 3d, 6a - 6k, 9a - 9e) zur direkten digitalen Erfassung eines Messwertes für den Parameter vorgesehen sind.

3. Vorrichtung nach Anspruch 1, **dadurch gekennzeichnet, dass** der Einzelsensor (5, 8) zur analogen Erfassung und mindestens ein Einzelsensor (6, 9) zur digitalen Erfassung eines Messwertes für einen Parameter mindestens teilweise demselben Bereich des Körpers zugeordnet sind.

4. Vorrichtung nach Anspruch 1, **dadurch gekennzeichnet, dass** dem Einzelsensor zur digitalen Erfassung Elemente (31, 32) zur Umwandlung seines Ausgangssignales in ein mindestens binäres Signal zugeordnet sind, wobei mindestens ein Schwellwert (54) vorgesehen ist.

5. Vorrichtung nach Anspruch 4, **dadurch gekennzeichnet, dass** die Elemente (31, 32) auf einer integrierten Schaltung mit dem Einzelsensor (30) integriert sind.

6. Vorrichtung nach Anspruch 1, **dadurch gekennzeichnet, dass** den Sensoren eine Lichtquelle (42) für gerichtetes Licht zugeordnet ist.

7. Vorrichtung nach Anspruch 1, **dadurch gekennzeichnet, dass** je ein optischer Sensor (10, 11) parallel zu einer ersten und zu einer zweiten zueinander geneigten Ebene (12, 13) angeordnet sind.

8. Verfahren zur optischen Erfassung von mindestens einem Parameter an einem längsbewegten fadenförmigen Körper (K), wobei ein Messwert für den Parameter digital erfasst wird, **dadurch gekennzeichnet, dass** vom Körper mindestens ein weiterer Messwert analog erfasst wird und dabei ein analoges und ein digitales Signal (57, 58) parallel erfasst werden.

9. Verfahren nach Anspruch 9, **dadurch gekennzeichnet, dass** ein Signal getaktet erfasst wird.

10. Verfahren nach Anspruch 9, **dadurch gekennzeichnet, dass** das getaktet erfasste Signal während einer vorgegebenen Zeit integriert und nach der vorgegebenen Zeit (48) zurückgesetzt wird.

11. Verfahren nach Anspruch 9, **dadurch gekennzeichnet, dass** das getaktet erfasste Signal laufend mit einem Schwellwert (54) verglichen wird und ein Ausgangssignal erzeugt wird, wenn der Schwellwert innerhalb der vorgegebenen Zeit erreicht wird.

12. Verfahren nach Anspruch 10, **dadurch gekennzeichnet, dass** die vorgegebene Zeit durch Vorgänge, die eine Lichtquelle (42) zur Beleuchtung des Köpers betreffen, beeinflusst und danach veränderbar ist.

## Claims

1. Device for the optical recording of at least one parameter on a longitudinally moved thread-type material (K) including an optical sensor (3, 4, 7) composed of at least two individual sensors (3a, 3b, 6a, 6b, 9a, 9b), in which at least one individual sensor is so constructed and arranged that one measured value is digitally recorded for the parameter, **characterised in that** at least one individual sensor (3e, 3f, 5, 8a, 8b) is provided in said sensor for the analog recording of a parameter.

2. Device according to claim 1, **characterised in that** a plurality of individual sensors (3a - 3d, 6a - 6k, 9a - 9e) are provided for the direct digital recording of a measured value for the parameter.

3. Device according to claim 1, **characterised in that** the individual sensor (5, 8) for the analog recording and at least one individual sensor (6, 9) for the digital recording of a measured value for a parameter are assigned at least partly to the same area of the material.

4. Device according to claim 1, **characterised in that** there are assigned to the individual sensor for the digital recording elements (31, 32) for converting its output signal into at least one binary signal, wherein at least one threshold value (54) is provided.

5. Device according to claim 4, **characterised in that** the elements (31, 32) are integrated on an integrated circuit with the individual sensor (30).

6. Device according to claim 1, **characterised in that** there is assigned to the sensors a light source (42) for directed light.

7. Device according to claim 1, **characterised in that** an optical sensor (10, 11) is disposed parallel to a first and to a second inclined plane (12, 13) respectively.

8. Method for the optical recording of at least one parameter on a longitudinally moved thread-type material (K), in which one measured value is digitally recorded for the parameter, **characterised in that** at least one more value for the parameter is recorded in analog manner and **in that** a analog signal and a digital signal (57, 58) are recorded in parallel.

9. Method according to claim 8, **characterised in that** one signal is recorded in clocked form.

10. Method according to claim 9, **characterised in that** the signal recorded in clocked form is integrated during a preset time and reset after the predetermined time (48).

11. Method according to claim 9, **characterised in that** the signal recorded in clocked form is compared continuously with a threshold value (54) and an output signal is generated if the threshold value is reached within the predetermined time.

12. Method according to claim 10, **characterised in that** the predetermined time is influenced by operations which concern a light source (42) for illuminating the material and is changeable thereafter.

## Revendications

1. Dispositif de détection optique d'au moins un paramètre sur un corps filiforme (K) se déplaçant dans le sens longitudinal avec un capteur optique (3, 4, 7) qui est composé d'au moins deux capteurs indépendants (3a, 3b, 6a, 6b, 9a, 9b), une valeur de mesure étant détectable numériquement pour les paramètres par au moins un capteur indépendant, **caractérisé en ce que** le capteur présente au moins un autre capteur indépendant (3e, 3f, 5, 8a, 8b) en vue de la détection analogique d'un paramètre.

2. Dispositif selon la revendication 1, **caractérisé en ce que** plusieurs capteurs indépendants (3a à 3d, 6a à 6k, 9a à 9e) sont prévus pour la détection numérique directe d'une valeur de mesure pour les paramètres.

3. Dispositif selon la revendication 1, **caractérisé en ce que** le capteur indépendant (5, 8) de détection analogique et au moins un capteur indépendant (6, 9) de détection numérique d'une valeur de mesure pour un paramètre sont affectés au moins partiellement à la même zone du corps.

4. Dispositif selon la revendication 1, **caractérisé en ce que** des éléments (31, 32) de transformation de son signal de sortie en un signal au moins binaire sont affectés au capteur indépendant de détection numérique, au moins une valeur de seuil (54) étant prévue.

5. Dispositif selon la revendication 4, **caractérisé en ce que** les éléments (31, 32) sont intégrés sur un circuit intégré avec le capteur indépendant (30).

6. Dispositif selon la revendication 1, **caractérisé en ce qu'**une source de lumière (42) pour lumière directe est affectée aux capteurs.

7. Dispositif selon la revendication 1, **caractérisé en ce qu'**un capteur optique (10, 11) est disposé à chaque fois parallèlement à un premier et à un second plans (12, 13) inclinés l'un par rapport à l'autre.

8. Procédé de détection optique d'au moins un paramètre sur un corps filiforme (K) se déplaçant dans le sens longitudinal, une valeur de mesure étant détectable numériquement pour les paramètres, **caractérisé en ce qu'**une autre valeur de mesure est détectée analogiquement du corps et ce faisant, un signal analogique et un signal numérique (57, 58) sont détectés parallèlement.

9. Procédé selon la revendication 9, **caractérisé en ce qu'**un signal est détecté cadencé.

10. Procédé selon la revendication 9, **caractérisé en ce que** le signal détecté cadencé est intégré durant un temps prédéfini et réinitialisé après le temps prédéfini (48).

11. Procédé selon la revendication 9, **caractérisé en ce que** le signal détecté cadencé est comparé en permanence à une valeur de seuil (54) et un signal de sortie est généré si la valeur de seuil est atteinte au cours du temps prédéfini.

12. Procédé selon la revendication 10, **caractérisé en ce que** le temps prédéfini est influencé, puis modifiable par les processus qui concernent une source de lumière (42) destinée à l'éclairage du corps.
